Europäisches Patentamt

European Patent Office

Office européen des brevets

(11) Veröffentlichungsnummer : **0 121 074**
**B1**

# (12) EUROPÄISCHE PATENTSCHRIFT

(45) Veröffentlichungstag der Patentschrift :
07.06.89

(51) Int. Cl.⁴ : **C 12 P 13/04**

(21) Anmeldenummer : 84101747.8

(22) Anmeldetag : 20.02.84

(54) Verfahren zur kontinuierlichen enzymatischen Umwandlung von alpha-Hydroxycarbonsäuren in entsprechende optisch aktive alpha-Aminocarbonsäuren.

(30) Priorität : 01.03.83 DE 3307094

(43) Veröffentlichungstag der Anmeldung :
10.10.84 Patentblatt 84/41

(45) Bekanntmachung des Hinweises auf die Patenterteilung : 07.06.89 Patentblatt 89/23

(84) Benannte Vertragsstaaten :
BE CH DE FR GB LI NL SE

(56) Entgegenhaltungen :
EP–A– 0 023 346
BIOTECH 84 - EUROPE, 1984, Seiten 391-404; C. WANDREY: "Production of L-amino acids from alpha-hydroxy acids"

(73) Patentinhaber : Degussa Aktiengesellschaft
Weissfrauenstrasse 9
D-6000 Frankfurt am Main 1 (DE)

Gesellschaft für biotechnologische Forschung mbH (GBF)
Mascheroder Weg 1
D-3300 Braunschweig-Stöckheim (DE)

Kernforschungsanlage Jülich Gesellschaft mit beschränkter Haftung
Postfach 1913
D-5170 Jülich 1 (DE)

(72) Erfinder : Leuchtenberger, Wolfgang, Dr. Dipl.-Chem.
Geschwister-Scholl-Strasse 1
D-6454 Bruchköbel (DE)
Erfinder : Wandrey, Christian, Prof. Dr.
Wolfshovener Strasse 139
D-5170 Jülich (DE)
Erfinder : Kula, Maria-Regina, Dr.
Forstweg 15
D-3340 Wolfsbüttel (DE)

EP 0 121 074 B1

## Beschreibung

Die Erfindung betrifft ein Verfahren zur kontinuierlichen enzymatischen Umwandlung von α-Hydroxycarbonsäuren in entsprechende optisch aktive α-Aminocarbonsäuren in einem Enzymreaktor in Gegenwart von durch Bindung an eine wasserlösliche hochmolekulare Substanz im Molekulargewicht vergrößertem Nicotinamid-adenin-dinucleotid (NAD+/NADH), einer für die eingesetzte α-Hydroxycarbonsäure spezifischen Dehydrogenase, einer für die zu bildende α-Aminocarbonsäure spezifischen Dehydrogenase und von Ammoniumionen.

Es ist bereits ein Verfahren zur kontinuierlichen Herstellung von Alanin aus Pyruvat in einem Enzymreaktor in Gegenwart von durch Bindung an ein wasserlösliches Dextran im Molekulargewicht vergrößertem Nicotinamid-adenin-dinucleotid (NAD+/NADH), von Lactat-Dehydrogenase, von Alanin-Dehydrogenase und von Ammoniumionen bekannt (K. Mosbach und P.-O. Larsson in Enzyme Engineering B III, Seiten 291 bis 298). Eigene Versuche haben jedoch ergeben, daß dieses bekannte Verfahren nicht über eine längere Zeitspanne kontinuierlich durchführbar ist, weil der Umsatz zu Alanin schon nach relativ kurzer Zeit stark abfällt, bis schließlich die Umsetzung völlig zum Stillstand kommt und überhaupt kein Alanin mehr gebildet wird.

Das erfindungsgemäße Verfahren ist nun dadurch gekennzeichnet, daß man einem mit einer Ultrafiltrationsmembran mit einer nominellen Ausschlußgrenze von 2.000 bis 50.000 ausgestatteten Membranreaktor, der eine wässerige Lösung von 0,1 bis 10 mmol/l an ein Polyethylenglykol mit einem durchschnittlichen Molekulargewicht zwischen 500 und 50.000 gebunden vorliegendem NAD+/NADH, der für die eingesetzte α-Hydroxycarbonsäure spezifischen Dehydrogenase und der für die zu bildende α-Aminocarbonsäure spezifischen Dehydrogenase enthält, kontinuierlich eine wässerige Lösung der umzusetzenden α-Hydroxycarbonsäure in einer 25 bis 100 % der maximal löslichen Menge entsprechenden Konzentration und der der α-Hydroxycarbonsäure entsprechenden α-Ketocarbonsäure in einer Konzentration zwischen 1 und 10 $k_m$, bezogen auf die eingesetzte Aminocarbonsäure-Dehydrogenase, und einer der umzusetzenden Menge an α-Hydroxycarbonsäure mindestens äquimolaren Menge an Ammoniumionen zuführt, über die Membran einen Differenzdruck zwischen 0,1 und 15 bar aufrechterhält und hinter der Membran kontinuierlich einen die gebildete α-Aminocarbonsäure enthaltenden Filtratstrom abführt.

Erst das erfindungsgemäße Verfahren gestattet es, α-Hydroxycarbonsäuren über eine längere Zeitspanne kontinuierlich in entsprechende optisch aktive α-Aminocarbonsäuren umzuwandeln. Die dafür wesentliche Maßnahme besteht darin, daß dem Membranreaktor kontinuierlich nicht nur die umzusetzende α-Hydroxycarbonsäure, sondern auch eine im Vergleich mit der letzteren wesentlich geringere Menge an der entsprechenden α-Ketocarbonsäure zugeführt wird. Mit Hilfe dieser Maßnahme ist es möglich, die eingesetzten α-Hydroxycarbonsäuren kontinuierlich und mit hohen Raum-Zeit-Ausbeuten in entsprechende optisch aktive α-Aminocarbonsäuren umzuwandeln und so diese α-Aminocarbonsäuren kostengünstig herzustellen.

Als Reaktionsgefäß wird ein mit einer Ultrafiltrationsmembran ausgestatteter Membranreaktor verwendet, dessen Membran dazu dient, die eingesetzten Enzyme und das für die Umsetzung erforderliche Coenzym im Reaktor zurückzuhalten, aber das niedermolekulare Produkt und das nicht umgesetzte Substrat durchzulassen. Der Membranreaktor kann als sogenannter Flachmembranreaktor ausgebildet sein. Bei diesem Reaktortyp kann es sich beispielsweise um ein flaches zylindrisches Gefäß handeln, auf das ein mittels eines O-Ringes abgedichteter Deckel aufgesetzt ist. Zusammen mit dem O-Ring ist die flächenmäßig relativ ausgedehnte flache Membran eingespannt. Der Substratstrom wird durch eine Dosierpumpe dem unterhalb der Membran liegenden Reaktionsraum zugeführt, der zweckmäßigerweise mit einer Rühreinrichtung, z. B. einem Magnetrührer, ausgestattet ist. Der das Produkt enthaltende Filtratstrom verläßt den Reaktionsraum durch die Membran und eine zwecks Vermeidung von deren mechanischer Beanspruchung darüber angeordnete, mit Bohrungen versehene Platte und wird aus dem Deckel abgeführt. Ein sogenannter Hohlfaser-Membranreaktor, in dem ein Hohlfaserbündel aus Ultrafiltrationsmembranen, ein sogenanntes Hohlfaser-Modul, an die Stelle der Flachmembran tritt, ist dann vorteilhafter, wenn sich aufgrund der geometrischen Anordnung höhere Renoldzahlen des Fluids parallel zur Membran und damit geringere Belegungen der Membran mit Enzymproteinen erreichen lassen. Bei diesem Reaktortyp handelt es sich beispielsweise um eine Art von Schlaufenreaktor, der aus einem Reaktionsbehälter, einer Umwalzpumpe und dem Hohlfaser-Modul besteht. Der Substratstrom wird mittels einer Dosierpumpe dem Reaktionsbehälter zugeführt. In diesem wird das Reaktionsgemisch umgepumpt, wobei der Umpumpstrom im Verhältnis zum Substratstrom mindestens etwa 100 : 1 beträgt, um die Belegung der Hohlfasermembranen mit Enzymprotein so gering wie möglich zu halten. Der das Produkt enthaltende Filtratstrom tritt durch die Hohlfasermembranen hindurch und wird hinter diesen gesammelt und abgeführt. Für das erfindungsgemäße Verfahren werden Membranen verwendet, die eine nominelle Ausschlußgrenze von 2.000 bis 50.000 aufweisen. Geeignete Materialien für die Membranen sind beispielsweise Acetylcellulosen, Polyamide, Polysulfone oder modifizierte Polyvinylalkohole.

Der Membranreaktor enthält eine wässerige Lösung von 0,1 bis 10 mmol/l an ein Polyethylenglykol mit einem durchschnittlichen Molekulargewicht zwischen 500 und 50.000, vorzugsweise zwischen 1.500 und 20.000, gebunden vorliegendem $NAD^+/NADH$, einer für die eingesetzte α-Hydroxycarbonsäure spezifischen Dehydrogenase und einer für die zu bildende α-Aminocarbonsäure spezifischen Dehydrogenase.

Das beim erfindungsgemäßen Verfahren als Coenzym erforderliche, durch Bindung an ein Polyethylenglykol im Molekulargewicht vergrößerte $NAD^+/NADH$ muß zwar noch wasserlöslich sein, um eine homogene Katalyse zu erlauben, andererseits aber zusammen mit den eingesetzten Enzymen durch die Membran sicher zurückgehalten werden. Ausführlich beschrieben ist die Herstellung von im Molekulargewicht vergrößertem $NAD^+/NADH$ beispielsweise in der DE-PS 28 41 414.

Zu diesem Zweck wird beispielsweise das Coenzym in seiner oxydierten Form zunächst mit Äthylenimin zum N(1)-Aminoethylderivat umgesetzt, welches dann seinerseits mit Hilfe der Carbodiimidmethode [vgl. Cuatrecanas, J. Biol. Chem., 245, 3059 (1970)] an ein carboxyliertes Polyethylenglykol gekuppelt wird, dessen Polyoxyethylenkette ein durchschnittliches Molekulargewicht zwischen 500 und 50.000, vorzugsweise zwischen 1.500 und 20.000, aufweist. Das erhaltene Kupplungsprodukt wird dann zum entsprechenden NADH-Derivat reduziert, durch eine Dimroth-Umlagerung in das N(6)-Derivat umgewandelt und gegebenenfalls wieder zum entsprechenden $NAD^+$-Derivat oxydiert. Das im Molekulargewicht vergrößerte Coenzym wird in einer solchen Menge eingesetzt, daß die Konzentration an $NAD^+/NADH$ 0,1 bis 10 mnol/l, vorzugsweise 1 bis 7 mmol/l, beträgt.

Dem Membranreaktor wird kontinuierlich eine wässrige Lösung der umzusetzenden α-Hydroxycarbonsäure in einer 25 bis 100 % der maximal löslichen Menge entsprechenden Konzentration zugeführt. Grundsätzlich ist es natürlich möglich, eine enantiomeren-reine α-Hydroxycarbonsäure, also entweder die L-Form oder die D-Form einzusetzen und im Membranreaktor eine für das eingesetzte Enantiomere spezifische Dehydrogenase vorzulegen.

Viel vorteilhafter ist es jedoch, die α-Hydroxycarbonsäure als Racemat einzusetzen. Dann bestehen bezüglich der zu verwendenden Dehydrogenase drei Möglichkeiten. Man kann eine Dehydrogenase einsetzen, die nur für das eine Enantiomere spezifisch ist. In diesem Falle bleibt das jeweils andere Enantiomere unverändert und verläßt den Membranreaktor mit dem Filtratstrom, aus dem es zurückgewonnen werden kann. Oder man setzt ein Gemisch aus einer für das L-Enantiomere und einer für das D-Enantiomere spezifischen Dehydrogenase ein. In diesem Falle werden dann beide Enantiomere letztlich in die gewünschte α-Aminocarbonsäure umgewandelt. Zum gleichen Ziel führt es aber auch, wenn man zwar nur eine für das L-Enantiomere oder das D-Enantiomere spezifische Dehydrogenase einsetzt, aber im Membranreaktor zusätzlich eine für die einge

setzte α-Hydroxycarbonsäure spezifische Racemase vorlegt.

Dem Membranreaktor wird ferner kontinuierlich die der umzusetzenden α-Hydroxycarbonsäure entsprechende α-Ketocarbonsäure in solcher Menge zugeführt, daß ihre Konzentration im Reaktor zwischen 1 und 10 $k_m$, bezogen auf die eingesetzte Aminocarbonsäure-Dehydrogenase, liegt. $k_m$ bedeutet hierbei den Kehrwert der Adsorptionsgleichgewichtskonstanten für die α-Ketocarbonsäure in Bezug auf die eingesetzte für die zu bildende α-Aminocarbonsäure spezifische Dehydrogenase.

Die α-Ketocarbonsäure kann entweder der wässerigen Lösung der umzusetzenden α-Hydroxycarbonsäure zugesetzt werden oder in Form einer wässrigen Lösung getrennt zudosiert werden, was unter Umständen vorteilhafter sein kann, weil sich dann die Dosierung unabhängig regeln läßt.

Dem Membranreaktor werden ferner kontinuierlich Ammoniumionen in einer der umzusetzenden Menge an α-Hydroxycarbonsäure mindestens äquimolaren Menge zugeführt. Aber auch ein bis zu 3-facher molarer Überschuß an Ammoniumionen stört die Umsetzung nicht. Die Zufuhr der Ammoniumionen erfolgt zweckmäßigerweise so, daß der wässerigen Lösung der umzusetzenden α-Hydroxycarbonsäure ein geeignetes Ammoniumsalz, beispielsweise Ammoniumformiat, in der gewünschten Menge zugesetzt wird.

Die für die umzusetzende α-Hydroxycarbonsäure spezifische Dehydrogenase wird zweckmäßig in einer solchen Menge eingesetzt, daß im Gleichgewichtszustand mindestens 50 % des erreichbaren Gleichgewichtsumsatzes in Bezug auf die als Zwischenprodukt auftretende entsprechende α-Ketocarbonsäure erzielt werden. Die erforderliche Enzymkonzentration hängt von der Verweilzeit und von der Ausgangskonzentration an α-Hydroxycarbonsäure ab. Bei einer Verweilzeit von 1 Stunde und einer Substratkonzentration von 100 mmol/l genügt im allgemeinen eine α-Hydroxycarbonsäure-Dehydrogenase-Aktivität von 10 U/ml.

Eine eventuell zusätzlich eingesetzte, für die umzusetzende α-Hydroxycarbonsäure spezische Racemase wird zweckmäßig in einer solchen Menge eingesetzt, daß die Racemisierungs-Reaktion mit ausreichender Geschwindigkeit verläuft und nicht zum geschwindigkeitsbestimmenden Schritt der gesamten Reaktion wird. Im allgemeinen genügt hierfür eine Racemase-Aktivität von 10 U/ml, wenn wiederum von einer Verweilzeit von 1 Stunde und einer Substratkonzentration von 100 mmol/l ausgegangen wird.

Die für die zu bildende α-Aminocarbonsäure spezifische Dehydrogenase schließlich wird zweckmäßig in einer solchen Menge eingesetzt, daß das sich im Gleichgewichtszustand einstellende Verhältnis von $NAD^+$ zu NADH mindestens 5 : 1 beträgt. Dazu werden bis zu 100 U/ml der Aminocarbonsäure-Dehydrogenase eingesetzt, um bei einer Verweilzeit von 1 Stunde und einer Substratkonzentration von 100 mmol/l minde

stens 90 % des Gleichgewichtsumsatzes, der mit dem gekoppelten Reaktionssystem thermodynamisch möglich ist, einzustellen.

Während der Umsetzung muß über die Membran ein Differenzdruck von 0,1 bis 15 bar, vorzugsweise von 0,2 bis 3 bar, aufrechterhalten werden, was durch Verwendung einer entsprechend dimensionierten Dosierpumpe für die zuzuführende Substratlösung und gegebenenfalls durch ein Drosselventil im Filtratstrom hinter der Membran erreicht wird. Der Differenzdruck bewirkt, daß ein Filtratstrom mit der gewünschten Geschwindigkeit durch die Membran hindurchtritt. Der Absolutdruck auf der Druckseite der Membran sollte zweckmäßigerweise so eingestellt werden, daß auch bei kräftigem Rühren oder Umpumpen im Reaktionsraum vor der Membran zur Erzeugung einer kräftigen Turbulenz längs der Membran und damit zur Vermeidung einer Belegung der Membran mit den Enzymen oder dem im Molekulargewicht vergrößerten Coenzym an keiner Stelle der Druck so weit abgesenkt wird, daß es zu einem Entgasen des Reaktionsgemisches auf der Druckseite kommt.

Der Membranreaktor wird auf einer für enzymatische Umsetzungen üblichen Temperatur zwischen 25 und 50 °C gehalten. Der pH des Reaktionsgemisches wird während der Umsetzung zweckmäßigerweise im Bereich zwischen 8 und 9,5 gehalten.

In der Praxis können derzeit nach dem erfindungsgemäßen Verfahren nur L-$\alpha$-Aminocarbonsäuren hergestellt werden, weil D-Aminosäure-Dehydrogenasen bisher nicht zur Verfügung stehen. Im übrigen aber ist das erfindungsgemäße Verfahren sehr vielseitig einsetzbar. Beispielsweise kann mit den Enzymsystemen L- oder D- oder L- und D-Lactat-Dehydrogenase + L-Alanin-Dehydrogenase oder L- oder D-Lactat-Dehydrogenase + Lactat-Racemase (E.C.5.1.2.1.) + L-Alanin-Dehydrogenase Milchsäure in L-Alanin umgewandelt werden. Mit dem Enzymsystem L- oder D- oder L-und D-2-Hydroxy-4-methylpentansäure-Dehydrogenase + L-Leucin-Dehydrogenase kann 2-Hydroxy-4-methylmercapto-buttersäure in L-Methionin, 2-Hydroxy-3-methylbuttersäure in L-Valin, 2-Hydroxy-4-methylpentansäure in L-Leucin oder 2-Hydroxy-3-methylpentansäure in L-Isoleucin umgewandelt werden. Mit dem Enzymsystem L- Oder D- oder L- und D-Lactat-Dehydrogenase + L-Phenylalanin-Dehydrogenase kann Phenylmilchsäure in L-Phenylalanin umgewandelt werden. Mit dem Enzymsystem L- oder D- oder L- und D-Indolyllactat-Dehydrogenase + L-Phenylalanin-Dehydrogenase kann Indolylmilchsäure in L-Tryptophan umgewandelt werden.

Das erfindungsgemäße Verfahren wird durch die nachfolgenden Beispiele näher erläutert :

Beispiel 1

Ein auf einer Temperatur von 25 °C gehaltener Flachmembranreaktor mit einem Volumen von 11,3 ml, der mit einem Magnetrührer und einer Ultrafiltrationsmembran von 62 mm Durchmesser mit einer nominellen Ausschlußgrenze von 5.000 (Lieferfirma : Amicon, Witten ; Typ YM5) ausgestattet war, wurde zur Sterilisation mittels einer auf einer Fördergeschwindigkeit von 11,3 ml pro Stunde eingestellten Dosierpumpe ca. Stunden lang mit wässriger 70 %iger Ethanol-Lösung durchgespült. Anschließend wurde während weiterer ca. 5 Stunden die wässrige Alkohollösung durch destilliertes Wasser verdrängt. Danach wurde mit einer Fördergeschwindigkeit von 9,3 ml pro Stunde ca. 5 Stunden lang eine über ein Sterilfilter (0,2 Vm) filtrierte Substratlösung zugeführt, die 243 mmol/l DL-Milchsäure in Form des Natriumsalzes und 486 mmol/l Ammoniumformiat enthielt und mit Natronlauge auf pH 9 eingestellt war. Gleichzeitig wurde mit einer zweiten Dosierpumpe über das gleiche Sterilfilter mit einer Fördergeschwindigkeit von 2 ml pro Stunde ca. 5 Stunden lang eine Pyruvatlösung zugeführt, die 11,3 mmol/l Brenztraubensäure in Form des Natriumsalzes enthielt und mit Natronlauge auf pH 9 eingestellt war. Dadurch ergab sich, solange die Reaktion im Reaktor noch nicht gestartet war, eine Ausgangskonzentration im Reaktor von 200 mmol/l DL-Lactat, 400 mmol/l Amnoniumformiat und 2 mmol/l Pyruvat. Danach wurde ein Enzym/Coenzym-Gemisch in die Substratdosierung über ein Septum vor dem Sterilfilter eingespritzt, derart, daß sich im Reaktor eine Konzentration von 1,32 mmol/l NAD$^+$/NADH, gebunden an ein Polyethylenglykol mit einem mittleren Molekulargewicht von 20.000 ergab, sowie eine L-Lactat-Dehydrogenase-Aktivität von 60,5 U/ml (gemessen mit 0,2 mol/l DL-Lactat, 2 mmol/l NAD$^+$, 25 °C pH 9) sowie eine L-Alanin-Dehydrogenase-Aktivität von 178,6 U/ml (gemessen mit 2 mmol/l Pyruvat 2 mmol/l NADH, 400 mmol/l Ammoniumformiat 25 °C, pH 9).

Der Reaktor wurde mit einer Verweilzeit von 60 Minuten bei 25 °C und pH 9 kontinuierlich über 6 Tage betrieben. Während der gesamten Betriebszeit betrug der Differenzdruck über die Membran ca. 1,7 bar. Der maximale Anfangsumsatz betrug 80 % und fiel im Laufe von 6 Tagen auf 60 % ab (Umsatz bezogen auf das L-Enantiomere im Substrat). Die maximale Raum-Zeit-Ausbeute betrug 1,92 mol/(lxd) bzw. 171 g/(lxd). Innerhalb einer Betriebszeit von 6 Tagen wurden 10,1 g bzw. 113 mmol L-Alanin erhalten. Die enzymatisch aktive Coenzymkonzentration fiel im Laufe von 6 Tagen von 1,32 mmol/l auf 0,49 mmol/l ab.

Pro mol Coenzym (verbraucht) wurden 12.000 mol Produkt gebildet. Das entspricht einem Bedarf von 615 mg NAD$^+$ (nativ)/kg Produkt.

Beispiel 2

Ein auf einer Temperatur von 25 °C gehaltener Flachmembranreaktor mit einem Volumen von 10,0 ml, der mit einem Magnetrührer und einer Ultrafiltrationsmembran von 62 mm Durchmesser mit einer nominellen Ausschlußgrenze von 5.000 (Lieferfirma : Amicon, Witten ; Typ YM5) ausgestattet war, wurde zur Sterilisation mittels einer auf eine Fördergeschwindigkeit von 20 ml/Stunde eingestellten Dosierpumpe ca. 2 Stunden lang mit 0,05 %iger wässriger Peressigsäurelösung durchgespült. Anschließend wurde wahrend weiterer ca. 10 Stunden die Sterilisationslösung durch destilliertes Wasser verdrängt. Danach wurde mit einer Fördergeschwindigkeit von 10 ml/Stunde 4 Stunden lang über einen Sterilfilter (0,2 μm) Substratlösung zugeführt, die 400 mmol/l DL-2-Hydroxy-4-methylmercapto-buttersäure (« DL-Hydroxymethionin »), 800 mmol/l Ammoniumformiat und 1 mmol/l 2-Keto-4-methylmercapto-buttersäure enthielt und mit Natronlauge auf pH 8 eingestellt war.

In einem getrennten Satzreaktor (Becherglas) ließ man 25 ml einer gleichkonzentrierten Substratlösung, die die Enzyme L-Leucin-Dehydrogenase und L-2-Hydroxy-4-methylpentansäure-Dehydrogenase sowie als Coenzym an Polyethylenglykol-20.000 gebundenes NADH enthielt, über 24 Stunden reagieren. Dabei wurden solche Enzym- und Coenzymkonzentrationen verwendet, daß im Laufe der 24 Stunden zumindest ein Umsatz erreicht wurde, wie er später im kontinuierlichen Versuch angestrebt werden sollte.

Diese Lösung wurde mit einer Fördergeschwindigkeit von 2,7 ml/Stunde nun in den Flachmembranreaktor eindosiert. Danach wurde mit gleicher Fördergeschwindigkeit die oben angegebene Substratlösung weiterdosiert.

Da die Enzyme und das Coenzym zwar den Sterilfilter vor dem Reaktor, nicht aber den Ultrafilter der Reaktoranordnung selbst passieren können, ergab sich im Reaktor eine Konzentration von 1,42 mmol/l Coenzym, sowie eine L-2-Hydroxy-4-methylpentansäure-Dehydrogenase-Aktivität von 4,0 U/ml (gemessen mit 0,4 mol/l DL-2-Hydroxy-4-methylmercapto-buttersäure, 0,1 mol/l Trispuffer, 1 mmol/l NAD$^+$, 25 °C, pH, 8) und eine L-Leucin-Dehydrogenase-Aktivität von 86,3 U/ml (gemessen mit 2 mmol/l 2-Keto-4-methylmercapto-buttersäure, 1 mmol/l NADH, 800 mmol/l Ammoniumformiat, 25 °C, pH 8).

Der Reaktor wurde mit einer Verweilzeit von 3,7 Stunden bei 25 °C und pH 8 unter den genannten Bedingungen kontinuierlich über ca. 4,5 Tage betrieben. Während dieser Betriebszeit betrug der maximale Differenzdruck über die Membran ca. 3 bar. Im Versuchszeitraum wurde ein maximaler Umsatz von 38,4 % erreicht, der sich im Laufe von 2,6 Tagen auf 36,4 % ermäßigte (Umsatz bezogen auf das L-Enantiomere im Substrat). Die maximale Raum-Zeit-Ausbeute betrug 0,5 mol/(lxd) bzw. 74,3 g/(lxd). Innerhalb einer Betriebszeit von 4,34 Tagen wurden 3 g bzw. 20 mmol L-Methionin erhalten. Die enzymatisch aktive Coenzymkonzentration fiel im Laufe von 4,34 Tagen von 1,42 mmol/l auf 1,25 mmol/l ab.

Pro mol Coenzym (verbraucht) wurden 11.800 mol Produkt gebildet. Das entspricht einem Bedarf von 378 mg NAD$^+$ (nativ)/kg Produkt.

## Patentansprüche

1. Verfahren zur kontinuierlichen enzymatischen Umwandlung von α-Hydroxycarbonsäuren in entsprechende optisch aktive α-Aminocarbonsäuren in einem Enzymreaktor in Gegenwart von durch Bindung an eine wasserlösliche hochmolekulare Substanz im Molekulargewicht vergrößertem Nicotin-amid-adenin-dinucleotid (NAD$^+$/NADH), einer für die eingesetzte α-Hydroxycarbonsäure spezifischen Dehydrogenase, einer für die zu bildende α-Aminocarbonsäure spezifischen Dehydrogenase und von Ammoniumionen, dadurch gekennzeichnet, daß man einem mit einer Ultrafiltrationsmembran mit einer nominellen Ausschlußgrenze von 2.000 bis 50.000 ausgestatteten Membranreaktor, der eine wässerige Lösung von 0,1 bis 10 mmol/l an ein Polyethylenglykol mit einem durchschnittlichen Molekulargewicht zwischen 500 und 50.000 gebunden vorliegendem NAD$^+$/NADH, der für die eingesetzte α-Hydroxycarbonsäure spezifischen Dehydrogenase und der für die zu bildende α-Aminocarbonsäure spezifischen Dehydrogenase enthält, kontinuierlich eine wässerige Lösung der umzusetzenden α-Hydroxycarbonsäure in einer 25 bis 100 % der maximal löslichen Menge entsprechenden Konzentration und der der α-Hydroxycarbonsäure entsprechenden α-Ketocarbonsäure in einer Konzentration zwischen 1 und 10 $k_m$, bezogen auf die eingesetzte Aminocarbonsäure-Dehydrogenase, und einer der umzusetzenden Menge an α-Hydroxycarbonsäure mindestens äquimolaren Menge an Ammoniumionen zuführt, über die Membran einen Differenzdruck zwischen 0,1 und 15 bar aufrechterhält und hinter der Membran kontinuierlich einen die gebildete α-Aminocarbonsäure enthaltenden Filtratstrom abführt.

2. Verfahren nach Anspruch 1, dadurch gekennzeichnet, daß man die umzusetzende α-Hydroxycarbonsäure als Racemat und ein Gemisch aus einer für das L-Enantiomere und einer für das D-Enantiomere spezifischen Dehydrogenase einsetzt.

3. Verfahren nach Anspruch 1, dadurch gekennzeichnet, daß man die umzusetzende α-Hydroxycarbonsäure als Racemat und nur eine für das L-Enantiomere oder das D-Enantiomere spezifische Dehydrogenase einsetzt, aber die Umsetzung in gleichzeitiger Gegenwart einer für die α-Hydroxycarbonsäure spezifischen Racemase vornimmt.

## Claims

1. A process for the continuous enzymatic conversion of α-hydroxycarboxylic acids into corresponding optically active α-aminocarboxylic acids in an enzyme reactor in the presence of nicotinamide adenine dinucleotide (NAD$^+$/NADH) increased in molecular weight by binding to a water-soluble substance of high molecular weight, a dehydrogenase specific to the α-hydroxycarboxylic acid used, a dehydrogenase specific to the α-aminocarboxylic acid to be formed and ammonium ions, characterized in that an aqueous solution of the α-hydroxycar-

boxylic acid to be reacted in a concentration corresponding to 25 to 100 % of the maximum soluble quantity and of the α-ketocarboxylic acid corresponding to the α-hydroxycarboxylic acid in a concentration of 1 to 10 $k_m$, based on the aminocarboxylic acid dehydrogenase used, and of a quantity of ammonium ions at least equimolar to the quantity of α-hydroxycarboxylic acid to be reacted is fed continuously to a membrane reactor which is equipped with an ultrafiltration membrane having a nominal exclusion limit of 2,000 to 50,000 and which contains an aqueous solution of 0.1 to 10 mmol/l of NAD+/NADH bound to a polyethylene glycol having an average molecular weight of 500 to 50,000, the dehydrogenase specific to the α-hydroxycarboxylic acid used and the dehydrogenase specific to the α-aminocarboxylic acid to be formed, a differential pressure of 0.1 to 15 bar is maintained across the membrane and a filtrate stream containing the α-aminocarboxylic acid formed is continuously run off behind the membrane.

2. A process as claimed in claim 1, characterized in that the α-hydroxycarboxylic acid to be reacted is used as racemate with a mixture of a dehydrogenase specific to the L-enantiomer and a dehydrogenase specific to the D-enantiomer.

3. A process as claimed in claim 1, characterized in that the α-hydroxycarboxylic acid to be reacted is used as racemate with only one dehydrogenase specific to the L-enantiomer or the D-enantiomer, but the reaction is carried out in the simultaneous presence of a racemase specific to the α-hydroxycarboxylic acid.

**Revendications**

1. Procédé pour la conversion enzymatique en continu d'acides α-hydroxycarboxyliques en acides α-aminocarboxyliques optiquement actifs correspondants, dans un réacteur enzymatique en présence de nicotinamide-adénine-dinucléotide (NAD+/NADH) à poids moléculaire accru par liaison à une substance à haut poids moléculaire, soluble dans l'eau, d'une déshydrogénase spécifique de l'acide α-hydroxycarboxylique utilisé, d'une déshydrogénase spécifique de l'acide α-aminocarboxylique à former, et d'ions ammonium, caractérisé en ce que l'on envoie en continu une solution aqueuse de l'acide α-hydroxycarboxylique à convertir, à une concentration correspondant à 25-100 % de la quantité soluble maximale, et de l'acide α-cétocarboxylique correspondant à l'acide α-hydroxycarboxylique, à une concentration comprise entre 1 et 10 km, par rapport à l'acide aminocarboxylique-déshydrogénase utilisée, et d'une quantité au moins équimolaire d'ions ammonium, par rapport à la quantité d'acide α-hydroxycarboxylique à convertir, à un réacteur à membrane muni d'une membrane à ultrafiltration ayant un seuil de coupure nominal de 2 000 à 50 000, qui contient une solution aqueuse de 0,1 à 10 mmoles/litre de NAD+/NADH se trouvant sous forme liée à un polyéthyléneglycol ayant un poids moléculaire moyen compris entre 500 et 50 000, de la déshydrogénase spécifique de l'acide α-hydroxycarboxylique utilisé, et de la déshydrogénase spécifique de l'acide α-aminocarboxylique à former, on maintient sur la membrane une pression différentielle comprise entre 0,1 et 15 bars, et en arrière de la membrane, on évacue en continu un courant de filtrat contenant l'acide α-aminocarboxylique formé.

2. Procédé selon la revendication 1, caractérisé en ce que l'on utilise sous forme de racémique l'acide α-hydroxycarboxylique à convertir, et un mélange d'une déshydrogénase spécifique de l'énantiomère L et d'une déshydrogénase spécifique de l'énantiomère D.

3. Procédé selon la revendication 1, caractérisé en ce que l'on utilise sous forme de racémique l'acide α-hydroxycarboxylique à convertir, et seulement une déshydrogénase spécifique de l'énantiomère L ou de l'énantiomère D, mais on effectue la conversion en présence simultanée d'une racémase spécifique de l'acide α-hydroxycarboxylique.